Europäisches Patentamt

European Patent Office

Office européen des brevets

(11) Publication number: **0 492 813 A2**

## EUROPEAN PATENT APPLICATION

(21) Application number: **91311057.3**

(22) Date of filing: **28.11.91**

(51) Int. Cl.5: **C07C 29/50**, C07C 31/04

(30) Priority: **21.12.90 GB 9027826**

(43) Date of publication of application:
**01.07.92 Bulletin 92/27**

(84) Designated Contracting States:
**AT BE CH DE DK ES FR GB GR IT LI LU NL SE**

(71) Applicant: **BP Chemicals Limited**
**Belgrave House 76 Buckingham Palace Road**
**London, SW1W 0SU(GB)**

(72) Inventor: **Elliott, Gregory Phillip**
**The British Petroleum Co. p.l.c., Chertsey**
**Road**
**Sunbury-on-Thames, Middlesex TW16**
**7LN(GB)**
Inventor: **Iamurri, Bertrand Gilbert**
**16 Avenue du Grand Gour Figuerolles**
**F-13500 Martigues(FR)**

(74) Representative: **Barlow, Michael Thomas et al**
**BP INTERNATIONAL LIMITED Patents &**
**Agreements Division Chertsey Road**
**Sunbury-on-Thames Middlesex TW16**
**7LN(GB)**

(54) **Process for oxidising alkanes to alcohols.**

(57) A process for the production of alcohols from alkanes comprises contacting a $C_1$ to $C_4$ alkane with an oxygen-containing gas at elevated temperature in the presence of a molybdenum oxide-containing catalyst which has been activated prior to use by contacting it with a $C_1$ to $C_4$ hydrocarbon at elevated temperature and pressure in the absence of a oxygen-containing gas.

This invention relates to a process for oxidising alkanes to alcohols and in particular ethane to methanol.

It is known to oxidise ethane to ethylene using catalysts based upon mixed oxides of molybdenum and vanadium. For example, US patent number US 4,524,236 describes a process for oxidising ethane to ethylene using a calcined oxide catalyst containing molybdenum, vanadium, niobium, antimony and at least one metal from a given group of metals

In US 4,524,236, the belief is stated that the catalyst according to that invention should have one or more of the metal components slightly below their highest possible oxidation states. A method of achieving this is described therein in which oxygen containing gas is passed over the catalyst during calcination to control the reducing actions of reducing agents which are introduced into the solution system from which the catalyst is prepared. A similar belief is described in US patents US 4,568,790 and US 4,596,787.

US patent US 4,339,355 describes a catalyst comprising the elements molybdenum, vanadium, niobium and at least one element selected from a given group for oxidising alpha-beta unsaturated aliphatic aldehydes to the corresponding acids. US 4,339,355 states the belief that for the catalyst of that invention to be most effective, the molybdenum, vanadium, niobium and X metal components should be slightly reduced below their highest possible oxidation states. According to US 4,379,355, this may be accomplished during the thermal treatment of the catalyst in the presence of reducing agents which are introduced into the solution system from which the catalyst is prepared. According to US 4,339,355, the catalyst may also be reduced in the reactor in which the oxidation reaction is to be conducted, by the passage of hydrogen or hydrocarbon reducing agents such as ethane, ethylene or propylene through the catalyst bed. US 4,339,355 only describes a process for oxidising alpha-beta unsaturated aliphatic aldehydes to produce alpha-beta unsaturated carboxylic acids. It makes no mention of a process for oxidising alkanes, in particular ethane.

US patent US 4,250,346 describes a process for catalytic oxydehydrogenation of ethane to ethylene using a catalyst comprising oxides of the elements $Mo_aX_bY_c$ as therein defined. US 4,250,346 states the belief that:

"for the catalysts to be most effective, the Mo, X and Y metal components should be slightly reduced below their highest possible oxidation states. This may be accomplished during the thermal treatment of the catalyst in the presence of reducing agents such as $NH_3$ or organic reducing agents, such as the organic complexing agents, which are introduced into the solution systems from which the catalysts are prepared. The catalyst may also be reduced in the reactors in which the oxidation reaction is to be conducted by the passage of hydrogen or hydrocarbon reducing agents such as ethane, ethylene or propylene through the catalyst bed."

However US 4,250,346 relates to a process for the production of ethylene and makes no mention of a process for producing methanol.

Oxidation of ethane with an oxgyen-containing gas over a catalyst based upon mixed oxide of molybdenum and vanadium generally produces, as principal products, ethylene and/or acetic acid. UK patent GB 1398385 relates to a process for the production of oxygenated hydrocarbons by partial oxidation under conditions chosen so that not less than 25 percent of the oxygen supplied issues from the reactor. In the examples a mixture of cupric oxide and molybdenum oxide is heated in air and used as catalyst. Using natural gas as reactant, useful products include methanol, formaldehyde acetaldehyde and ethylene. According to GB 1398385, a considerable proportion of the oxygen supplied issues unconsumed from the reactor. When oxygen consumption was allowed to increase carbon monoxide and ethylene formation were increased; the latter being a less preferred product than aldehydes or methanol. Activation of the catalyst in the absence of oxygen is not described.

There remains a need for a process to produce alcohols by the oxidation of alkanes with an oxygen-containing gas over a catalyst based upon molybdenum oxide.

Thus, according to the present invention there is provided a process for the oxidation of alkanes by contacting a $C_1$ to $C_4$ alkane with an oxygen-containing gas at elevated temperature in the presence as catalyst of a composition comprising molybdenum in combination with oxygen characterised in that the catalyst is activated prior to use by contacting a $C_1$ to $C_4$ hydrocarbon with the catalyst composition in the absence of an oxygen-containing gas under conditions of elevated temperature and pressure suitable to activate the catalyst for the oxidation of the alkane to produce alcohol.

It has been found in the present invention that by activating the catalyst composition with a $C_1$ to $C_4$ hydrocarbon at elevated temperature and pressure in the absence of oxygen, the reaction products of the subsequent alkane oxidation process comprises significant quantities of alcohol. Thus for example, the oxidation products for ethane are not predominantly ethylene and/or acetic acid but instead comprise significant quantities of methanol together with carbon monoxide and methane with lesser amounts of ethanol, acetadehyde and carbon dioxide.

A catalyst composition suitable for the present invention may comprise oxides of the elements Mo, X and $X^1$ wherein

X = Cr, Mn, Nb, Ta, Ti, V and/or W;

$X^1$ = Bi, Ce, Co, Cu, Fe, K, Mg, Ni, P, Pb, Sb, Si, Sn, Tl and/or U; and the relative gram-atom ratios of the elements Mo:X:$X^1$ are a:b:c;

wherein a = 1, b = 0 to 2, c = 0 to 2 and preferably with the proviso that the total value of c for Co, Ni and/or Fe is <0.5. The preparation of such a catalyst composition is described in US 4,250,346.

Preferably, the catalyst composition comprises oxides of molybdenum and vanadium. More preferably, such a catalyst composition comprises the elements Mo, V, Nb, Sb and Z in the form of oxides in which the relative gram-atom ratios of Mo:V:Nb:Sb:Z are e:f:g:h:i wherein Z = at least one of the elements Li, Sc, Na, Be, Mg, Ca, Sr, Ba, Ti, Zr, Hf, Y, Ta, Cr, Fe, Co, Ni, Ce, La, Zn, Cd, Hg, Al, Tl, Pb, As, Bi, Te, U, K, Rb and W, preferably alkali metal or calcium and e = 0.5 to 0.9;

f = 0 to 0.4, preferably 0.1 to 0.4;

g = 0.001 to 0.2;

h = 0 to 0.1, preferably 0.001 to 0.1;

i = zero or 0.001 to 1.0.

The preparation of such a catalyst composition is described in US patents US 4,524,236, US 4,596,787 and US 4,568,790.

An example of such a catalyst composition suitable for use in the process of the present invention is:

$$Mo_{0.62}V_{0.26}Hb_{0.07}Sb_{0.02}Ca_{0.03} \qquad (I)$$

It will be understood that the elements are present in combination with oxygen.

Another catalyst composition suitable for use in the present invention comprises oxides of the elements Mo, V, Nb and F in the ratio Mo:V:Nb:F = s:t:u:v wherein

F is Co, Cr, Cu, Fe, In, Mn and/or Y,

s is 12,

t is 0.1 to 20 preferably 1 to 14 and most preferably 2 to 8,

u is 0.1 to 12 and preferably 0.5 to 2 and

v is 0 to 3.0 and preferably 0.01 to 1.0.

The numerical values of s, t, u and v represent the relative gram-atoms ratios of the elements Mo, V, Nb and F respectively, which are present in the catalyst composition in combination with oxygen. Any portion and preferably about less than 50% by weight of the niobium may be replaced by titanium and/or tantalum. The preparation of such catalyst composition is described in US patent US 4,339,355.

A catalyst composition suitable for use in the present invention may also comprise a molybdenum - containing ethane oxidative dehydrogenation catalyst composition in which the molybdenum is replaced in part by either rhenium or a combination of rhenium and tungsten. Suitably such a catalyst composition comprises the elements A, Q and R in combination with oxygen, the gram-atom ratios of the elements A:Q:R being j:k:l,

wherein

A = $Mo_mRe_nW_p$,

Q = Cr, Mn, Nb, Ta, Ti, V and/or W, and preferably Mn, Nb, V and/or W,

R = Bi, Ce, Co, Cu, Fe, K, Mg, Ni, P, Pb, Sb, Si, Sn, Tl and/or U, and preferably Sb, Ce and/or U,

j = 1,

k = 0 to 2, preferably 0.05 to 1.0,

l = 0 to 2, preferably 0.001 to 1.0, and more preferably 0.05 to 1.0 with the proviso that the total value of 1 for Co, Ni, and/or Fe is less than 0.5,

m + n + p = j,

m is greater than zero,

n is greater than zero, and

p is either zero or greater than zero.

A preferred such a catalyst composition comprises the elements A, Nb, Sb and T in combination with oxygen, the gram-atom ratios of the elements A:Nb:Sb:T being j:k:l:q,

wherein

A is the same as hereinbefore defined,

T is at least one of Li, Sc, Na, Be, Mg, Ca, Sr, Ba, Ti, Zr, Hf, Y, Ta, Cr, Fe, Co, Ni, Ce, La, Zn, Cd, Hg,

3

Al, Tl, Pb, As, Bi, Te, U, K, Rb and W, preferably alkali metal or calcium,

j,k and l are the same as hereinbefore defined, and

q is 0 to 2, preferably greater than zero.

A more preferred catalyst composition of this type comprises the elements A, V, Nb, Sb and T in combination with oxygen, the gram-atom ratios of the elements A:V:Hb:Sb:T being j:r:k:l:q wherein T and A are the same as hereinbefore defined,

j,k,l and q are the same as hereinbefore defined, and

r is 0 to 1.0.

Another catalyst suitable for use in the present invention comprises of oxides of the elements A, V, Nb and F in the ratio A:V:Nb:F = s:t:u:v

wherein A = $Mo_x Re_y W_z$ such that $x+y+z = s$ and x is greater than zero, y is greater than zero and z is either zero or greater than zero, and F, s, t, u and v are as hereinbefore defined. Any portion and preferably about less than 50% by weight of the niobium may be replaced by titanium and/or tantalum. Such catalysts may be prepared by procedures similar to those described in US 4,339,355.

Examples of catalysts suitable for use in the process of the present invention include:

$$Mo_{0.56} Re_{0.06} V_{0.26} Nb_{0.07} Sb_{0.03} Ca_{0.02} \qquad \text{(II)}$$

$$Mo_{0.37} Re_{0.25} V_{0.26} Nb_{0.07} Sb_{0.03} Ca_{0.02} \qquad \text{(III)}$$

$$Mo_{0.24} Re_{0.37} V_{0.26} Nb_{0.07} Sb_{0.04} Ca_{0.02}, \text{ and} \qquad \text{(IV)}$$

It will be understood that the elements are present in combination with oxygen. The preparation of such catalysts is described in our pending European Patent Publication No. EP-A-0407091.

The catalyst composition may be supported on a support. Suitable supports include silica, alumina, silicon carbide, zirconia, titania and mixtures thereof. When supported, the catalyst composition is usually present at 5 to 50% by weight.

The catalyst composition may be prepared by any of the methods conventionally employed for the preparation of catalysts. Suitably the catalyst composition may be prepared from a solution of soluble compounds and/or complexes and/or compounds of each of the elements. The solution is preferably an aqueous system having a pH in the range from 1 to 12, preferably from 2 to 8, at a temperature of from 20° to 100°C.

Generally, a mixture of compounds containing the elements is prepared by dissolving sufficient quantities of soluble compounds and dispersing any insoluble compounds so as to provide a desired gram-atom ratio of the elements in the catalyst composition. The catalyst composition may then be prepared by removing the solvent from the mixture. The catalyst may be calcined by heating to a temperature of from 200 to 550°C, suitably in air to oxygen, for a period of from 1 minute to 24 hours. Preferably, the air or oxygen is slowly flowing.

Further details of suitable methods for preparing the catalyst composition may be found in, for example US 4,596,787, US 4,568,790, US 4,524,236 and US 4,250,346.

Suitably, the catalyst composition is activated by contacting with the $C_1$ to $C_4$ hydrocarbon at an elevated temperature of about 200°C to about 550°C, preferably at about 250°C to about 450°C and an elevated pressure of about 5 barg to about 35 barg, preferably at about 10 barg to about 28 barg for a period of up to about 24 hours, preferably up to about 3 hours, more preferably up to about 90 minutes.

The catalyst composition is heated to the elevated temperature in the presence of the $C_1$ to $C_4$ hydrocarbon at a rate in the range 0.1 to 100°C/min, preferably about 4 to 10°C/min, more preferably about 6°C/min.

The $C_1$ to $C_4$ hydrocarbon used for activating the catalyst composition preferably comprises substantially pure ethane, propane and/or ethylene. The $C_1$ to $C_4$ hydrocarbon may be the same as the gas feedstock for the $C_1$ to $C_4$ alkane oxidation process.

In the alkane oxidation process the catalyst may be used in the form of a fixed or a fluidised bed.

In the alkane oxidation process, the alkane is preferably ethane which may be used in substantially pure form or admixed with one or more of nitrogen, methane, carbon dioxide and water in the form of steam, which may be present in major amounts, for example greater than 5 volume percent or one or more of hydrogen, carbon monoxide, $C_3/C_4$ alkanes and alkenes, which may be present in minor amounts, for example less than 5 volume percent.

In the alkane oxidation process the molecular oxygen-containing gas may be air or a gas richer or poorer in molecular oxygen than air, for example oxygen. A suitable gas may be, for example, oxygen diluted with a suitable diluent, for example nitrogen or helium.

Preferred concentrations in the gas feed to the alkane oxidation process are about 20 to 70% alkane, and about 6 to 8% oxygen.

The elevated temperature for the alkane oxidation process may suitably be in the range from about 200 to about 500°C, preferably from about 200 to about 400°C.

The pressure for the alkane oxidation process may suitably be atmosperic or superatmospheric, for example in the range from about 1 to about 50 bar, preferably from about 1 to about 30 bar.

The process of the invention will now be further illustrated by reference to the following Examples. In the Examples the following terms are used:

$$GHSV = \text{Gas Hourly Space Velocity}$$

$$= \frac{\text{Volume of gas flowing through catalyst bed (ml/hr)}}{\text{Volume of catalyst bed (ml).}}$$

$$\text{Ethane conversion (\%)} = 100 \times \frac{S}{S + [C_2H_6]}$$

where $S = [CO]/2 + [CO_2]/2 + [C_2H_4] + [CH_3COOH] + [CH_3OH]/2 + [C_2H_5OH] + [CH_4]/2 + [C_2H_4O]$

$$\text{Ethylene selectivity (\%)} = 100 \times \frac{[C_2H_4]}{S}$$

$$\text{Acetic acid selectivity (1\%)} = 100 \times \frac{[CH_3COOH]}{S}$$

$$\text{Methanol selectivity (\%)} = 100 \times \frac{[CH_3OH]/2}{S}$$

$$\text{CO selectivity (\%)} = 100 \times \frac{[CO]/2}{S}$$

$$\text{CO}_2 \text{ selectivity (\%)} = 100 \times \frac{[CO_2]/2}{S}$$

$$\text{Ethanol selectivity (\%)} = 100 \times \frac{[C_2H_5OH]}{S}$$

$$\text{Acetaldehyde selectivity (\%)} = 100 \times \frac{[C_2H_4O]}{S}$$

$$\text{Methane selectivity (\%)} = 100 \times \frac{[CH_4]/2}{S}$$

wherein [ ] = concentrations in mol %
and $[C_2H_6]$ = concentration of unconverted ethane.

CATALYST PREPARATION

In the Examples, the catalyst compositions are expressed in terms of relative gram-atoms of elements. It will be understood that the elements are present in combination with oxygen.

CATALYST (I)

A catalyst was prepared with the following composition:
$Mo_{0\,62}\,V_{0\,26}\,Nb_{0\,07}\,Sb_{0\,02}\,Ca_{0\,03}$,
the elements being present in combination with oxygen.

A first mixture, A was prepared by dissolving ammonium vanadate in deionised water in a beaker. A second mixture, B was prepared by partially dissolving niobium oxalate in deionised water in a beaker. To this was added antimony acetate and calcium nitrate together with more deionised water. A third mixture, C was prepared by dissolving ammonium molybdate in deionised water. Mixtures A and B were heated separately with stirring for 15 minutes to between 50 and 80°C. Both beakers contained undissolved solid and/or suspension after this time. Mixture A was added to mixture B and the whole heated with stirring for 15 minutes. During this time mixture C was heated with stirring to 50-80°C. After mixture C and the combination of mixtures A and B had been heated for 15 minutes, mixture C was added to the combination of mixtures A and B which was about 70°C. The whole was heated and stirred for a further 15 minutes at about 70°C before being brought to boiling point and the water evaporated off to leave a slurry. The slurry was dried at 115°C for 16 hours in an oven. The material was then sieved to 2.0mm to 0.18mm before being heated at 2 degress/min to 350°C under a flow of air (5-10ml/minute) and calcined at about 400°C for 4 hours. The air flow was then stopped and the material cooled. The product catalyst composition was sieved to 500 - 180 microns for use in the catalyst testing procedures.

CATALYST TESTING METHOD

3ml of the catalyst was loaded into a corrosion resistant stainless steel tubular reactor of internal diameter 5.6 mm and the reactor was placed in a tubular furnace.

The reactor was provided with a feed-gas manifold to allow various gases to be passed as required through a preheater zone at 200°C and then over the catalyst. Pressure in the reactor was controllable by a back-pressure regulator.

A pump was provided for optionally introducing water through the preheater zone where it was vapourised and mixed with the feed gas before passing over the catalyst. The reactor temperature was monitored by means of a thermocouple in the catalyst bed. The product vapours and gases leaving the reactor were sampled and analysed by gas-liquid chromatography (GLC).

GLC SPECIFICATION : Gas analysis : 3m Carbosieve S2 column and molecular sieve column.
Liquid analysis : 2.5m CarboPak B/Peg 20M column.

Example 1

A 3 ml sample of Catalyst I was placed in the reactor and brought to pressure (24 barg) with an ethane (99.9% pure) feed gas at 25°C.

To activate the catalyst, the temperature of the reactor was then raised to 270°C at a rate of 6°C/min. using the furnace with the flow of ethane continuing at a flow rate up to 12 1/hr.

When the reactor was at 270°C a feed gas comprising 70 volume % ethane, 7 volume % oxygen and balance helium was fed to the reactor at 6 to 12 1/hr in place of the ethane.

The activity of the catalyst at total oxygen consumption was at least (being limited by the rate at which oxygen could be introduced to the reactor) 75 ml catalyst/mole $O_2$/hr ie 1.6 times greater than that in Comparative Experiment 1, below. Catalyst activity is the amount (ml) of catalyst required to consume 1 mole of oxygen per hour at total oxygen consumption.

Selectivities to the various products are given in Table 1 below.

EP 0 492 813 A2

TABLE 1

| Product | Selectivities |
|---|---|
| Methanol | 20% |
| Carbon Monoxide | 38 - 40% |
| Carbon Dioxide | 3 - 4% |
| Ethylene | 14% |
| Acetaldehyde | 4 - 5% |
| Methane | 14 - 15% |
| Ethanol | 3 - 4%. |

It was found that the pressure in the reactor could be reduced to 5 barg and the temperature to 120°C (together or independently) and the above products were still produced in about the same selectivities.

Reducing the pressure below 5 barg and the temperature below 120°C (either independently or together) caused the oxidation reaction to cease.

Cofeeding water at 0.27g/l of gas in the stream at 25 barg and 270° to 330°C did not affect the activity and only had a very little effect on the selectivities.

Comparative Experiment 1

This experiment is not according to the present invention.

A 3 ml sample of Catalyst I was placed in a reactor similar to that for Example 1 and a flow of helium/oxygen (71%/29%) gas was passed through the reactor.

To activate the catalyst in accordance with conventional procedures, the temperature of the reactor was raised by the furnace to 280°C at a rate of 6°C/min.

When the reactor was at 280°C a feed gas comprising 20 volume % ethane, 7 volume % oxygen and balance helium was passed through the reactor at 6 to 12 litres/hour and the pressure raised to 14 barg. Water feed, as required was introduced to the preheater at 0.27g/litre of gas in the stream prior to raising the pressure.

Catalyst activity was measured at approaching total oxygen consumption to be 123 ml catalyst/mole $O_2$/hr. Catalyst activity is the amount (ml) of catalyst required to consume 1 mole of oxygen per hour at near total oxygen consumption.

Product selectivities are given in Table 2 below.

TABLE 2

| Product Selectivities | | |
|---|---|---|
| Selectivities to | With Water Cofeed | Without Water Cofeed |
| Acetic Acid | 26 % | 13 % |
| Acetic Acid & Ethylene | 86 % | 85 % |

No reaction was observed below 270°C reactor temperature.

Example 2

An experiment was performed using the same apparatus as in Example 1 and with a similarly prepared catalyst in the following steps:

1. The catalyst was heated to 250°C, 24 barg under ethane to activate it according to the invention.

2. The feed gas was changed to ethane 70 vol.%, oxygen 1 vol.%,balance helium. No exotherm was observed. Analysis of the products showed carbon monoxide (major gaseous product) methane, carbon dioxide and ethylene with methanol (major liquid product present in gas product stream as vapour) acetaldehyde and ethanol. This product distribution did not vary with changes in the flow rate of feed gas through the reactor ie different Gas Hourly Space Velocities (GHSV).

3. The feed gas was changed to a helium/oxygen mixture and the catalyst was heated at 350°C and 24 barg for about 15 hours (conventional catalyst activation procedure).

4. The catalyst was cooled to 50°C and step (1) was repeated, followed by step (2) but at 270°C. The same product distributions as in step (2) were obtained over 15 hours.

5. The feed gas was changed to one comprising oxygen 15%, helium 55% and ethane 30% with resulting exotherms in the catalyst up to 400°C for 30 minutes.

6. The feed gas was then returned to that of step (2) and at 300°C. Product selectivities were the same as step (2) over 30 hours despite changing the feed gas to a mixture comprising ethane 30%, oxygen 7% and helium balance after 15 hours.

7. The reactor temperature was decreased to 50°C and the pressure reduced to 14 barg.

8. Then the reactor temperature was increased to 250°C and gc analysis showed the same product selectivites as in step (2).

9. The reactor pressure was reduced to atmospheric and the reactor temperature was reduced to 50°C.

10. The reactor temperature was increased in stages to 280°C at atmospheric pressure. No reaction was detected by gc analysis with a feed gas of ethane 70%, oxygen 6.3%, balance helium. This shows that ethane oxidation only takes place in this temperature region at elevated pressure.

11. The reactor pressure was then increased to 10 barg with a small increase in temperature (4°C) and gc analysis indicated conventional product selectivites to ethylene and acetic acid rather than methanol. This shows that heating the catalyst in step 10 at atmospheric pressure in the presence of an ethane-and-oxygen-containing gas has caused it to change to a catalyst which gives ethylene and acetic acid oxidation products.

12. Increasing the pressure to 14 barg and the temperature to 300°C increased the amount of ethylene and acetic acid over that produced in step (11) and traces of carbon dioxide were formed.

## Claims

1. A process for the oxidation of alkanes by contacting a $C_1$ to $C_4$ alkane with an oxygen-containing gas at elevated temperature in the presence as catalyst of a composition comprising molybdenum in combination with oxygen characterised in that the catalyst is activated prior to use by contacting a $C_1$ to $C_4$ hydrocarbon with the catalyst composition in the absence of an oxygen-containing gas under conditions of elevated temperature and pressure suitable to activate the catalyst for the oxidation of the alkane to produce an alcohol.

2. A process as claimed in claim 1 characterised in that the catalyst composition comprises oxides of the elements Mo, X and $X^1$ wherein

    X =     Cr, Mn, Hb, Ta, Ti, V and/or W

    $X^1$ =     Bi, Ce, Co, Cu, Fe, K, Mg, Ni, P, Pb, Sb, Si, Sn, Tl and/or U;

and the relative gram-atom ratios of the elements Mo:X:$X^1$ are a:b:c wherein a = 1, b = 0 to 2, c = 0 to 2.

3. A process as claimed in claim 1 characterised in that the catalyst composition comprises oxides of molybdenum and vanadium.

4. A process as claimed in claim 3 characterised in that the catalyst composition comprises the elements Mo, V, Nb, Sb and Z in the form of oxides in which the relative gram-atom ratios of Mo:V:Nb:Sb:Z are e:f:g:h:i wherein Z = at least one of the elements Li, Sc, Na, Be, Mg, Ca, Sr, Ba, Ti, Zr, Hf, Y, Ta, Cr, Fe, Co, Ni, Ce, La, Zn, Cd, Hg, Al, Tl, Pb, As, Bi, Te, U, K, Rb and W,

    e =     0.5 to 0.9;

    f =     0 to 0.4,

    g =     0.001 to 0.2;

    h =     0 to 0.1,

    i =     zero or 0.001 to 1.0.

5. A process as claimed in claim 3 characterised in that the catalyst composition comprises oxides of the element Mo, V, Nb, and F in the ratio Mo:V:Nb:F = s:t:u:v wherein

    F is     Co, Cr, Cu, Fe, In, Mn and/or Y,

    s is     12,

    t is     0.1 to 20,

    u is     0.1 to 12,

v is      0 to 3.0,

the numerical values s, t, u and v representing the relative gram-atom ratios of the elements Mo, V, Nb and F respectively which are present in the catalyst composition in combination with oxygen.

6. A process as claimed in claim 5 characterised in that about less than 50% by weight of the niobium is replaced by titanium and/or tantalum.

7. A process as claimed in claim 1 characterised in that the molybdenum of the catalyst composition has been replaced in part by rhenium or a combination of rhenium and tungsten.

8. A process as claimed in claim 7 characterised in that the catalyst composition comprises the elements A, Q and R in combination with oxygen the gram-atom ratios of the elements A:Q:R being j k:l, wherein

| | |
|---|---|
| A = | $Mo_m Re_n Wp$, |
| Q = | Cr, Mn, Nb, Ta, Ti, V and/or W, |
| R = | Bi, Ce, Co, Cu, Fe, K, Mg, Ni, P, Pb, Sb, Si, Sn, Tl and/or U, |
| j = | 1, |
| k = | 0 to 2, |
| l = | 0 to 2, |
| m + n + p = | j, |
| m is | greater than zero, |
| n is | greater than zero, and |
| p is | either zero or greater than zero. |

9. A process as claimed in claim 7 characterised in that the catalyst composition comprises the elements A, Nb, Sb and T in combination with oxygen, the gram-atom ratios of the elements A:Nb:Sb:T being j:k:l:q, wherein

A =        $Mo_m Re_n W_p$,

T is at least one of Li, Sc, Na, Be, Mg, Ca, Sr, Ba, Ti, Zr, Hf, Y, Ta, Cr, Fe, Co, Ni, Ce, La, Zn, Cd, Hg, Al, Tl, Pb, As, Bi, Te, U, K, Rb and W,

| | |
|---|---|
| j = | 1, |
| k = | 0 to 2, |
| l = | 0 to 2, |
| m + n + p = | j |
| m is | greater than zero, |
| n is | greater than zero, |
| p is | either zero or greater than zero, and |
| q is | 0 to 2, preferably greater than zero. |

10. A process as claimed in claim 7 characterised in that the catalyst composition comprises the elements A, V, Nb, Sb and T in combination with oxygen, the gram-atom ratios of the elements A:V:Nb:Sb:T being j:r:k:l:q wherein

A =        $Mo_m Re_n W_p$,

T is at least one of Li, Sc, Na, Be, Mg, Ca, Sr, Ba, Ti, Zr, Hf, Y, Ta, Cr, Fe, Co, Ni, Ce, La, Zn, Cd, Hg, Al, Tl, Pb, As, Bi, Te, U, K, Rb and W,

| | |
|---|---|
| j = | 1 |
| k = | 0 to 2, |
| l = | 0 to 2, |
| m + n + p = | j, |
| m is | greater than zero, |
| n is | greater than zero, |
| p is | either zero or greater than zero, |
| q is | 0 to 2, preferably greater than zero, and |
| r is | 0 to 1.0. |

11. A process as claimed in claim 7 characterised in that the catalyst composition comprises oxides of the elements A, V, Nb and F in the ratio A:V:Nb:F = s:t:u:v wherein

A =                $Mo_x Re_y W_z$,

F is            Co, Cr, Cu, Fe, In, Mn and/or Y,
$x + y + z =$    s,
x is            greater than zero,
y is            greater than zero,
z is            either zero or greater than zero,
s =            12,
t is            0.1 to 20,
u is            0.1 to 12,
v is            0 to 3.0

the numerical values of s, t, u, v, x, y and z representing the relative gram-atom ratios of the elements which are present in the catalyst composition in combination with oxygen.

**12.** A process as claimed in claim 1 characterised in that the catalyst composition has an empirical formula selected from the group consisting of:

$$Mo_{0.62} \; V_{0.26} \; Nb_{0.07} \; Sb_{0.02} \; Ca_{0.03} \qquad (I)$$

$$Mo_{0.56} \; Re_{0.06} \; V_{0.26} \; Nb_{0.07} \; Sb_{0.03} \; Ca_{0.02} \qquad (II)$$

$$Mo_{0.37} \; Re_{0.25} \; V_{0.26} \; Nb_{0.07} \; Sb_{0.03} \; Ca_{0.02} \qquad (III) \; and$$

$$Mo_{0.24} \; Re_{0.37} \; V_{0.26} \; Nb_{0.07} \; Sb_{0.04} \; Ca_{0.02} \qquad (IV),$$

the elements being present in combination with oxygen.

**13.** A process as claimed in any one of the preceding claims characterised in that the $C_1$ to $C_4$ alkane reactant comprises ethane and the alcohol product comprises methanol.

**14.** A process as claimed in any one of the preceding claims characterised in that the catalyst composition is activated by contacting with ethane, propane and/or ethylene.

**15.** A process as claimed in claim 13 characterised in that the activating hydrocarbon comprises ethane.

**16.** A process as claimed in any one of the preceding claims characterised in that the catalyst composition is activated at an elevated temperature of about 200 to about 550°C and an elevated pressure of about 5 barg to about 35 barg for a period of up to about 24 hours.

**17.** A process for the production of methanol from ethane characterised in that the process comprises the steps of (1) activating a catalyst composition comprising molybdenum in combination with oxygen, by contacting ethane in the absence of oxygen with the catalyst composition at an elevated temperature of about 200°C to about 550°C and an elevated pressure of about 5 barg to about 35 barg for a period of up to about 24 hours to activate the catalyst composition for the oxidation reaction and (2) contacting ethane and an oxygen-containing gas with the activated catalyst at an elevated temperature of from about 200°C to about 500°C and a pressure in the range from about 1 bar to about 50 bar.